# EUROPEAN PATENT SPECIFICATION

(11) **EP 3 160 244 B1**
(45) Date of publication and mention of the grant of the patent: **07.03.2018**
(21) Application number: 15735653.6
(22) Date of filing: 29.06.2015
(51) Int. Cl.: A23C 3/00, A23C 7/00, A61L 2/00, B65B 55/02, B67C 7/00

(54) **A METHOD FOR EFFICIENTLY EMPTYING A SYSTEM WITH LIQUID PRODUCT**
VERFAHREN ZUR EFFIZIENTEN ENTLEERUNG EINES SYSTEMS MIT EINEM FLÜSSIGPRODUKT
PROCÉDÉ POUR VIDER EFFICACEMENT UN SYSTÈME AVEC UN PRODUIT LIQUIDE

(30) Priority: 30.06.2014 SE 1450808
(43) Date of publication of application: 03.05.2017
(73) Proprietor: Tetra Laval Holdings & Finance SA, 1009 Pully (CH)
(72) Inventor: ANDERSSON, Göran, S-243 72 Tjörnarp (SE); OLSSON, Bo, S-21580 Malmö (SE)
(74) Representative: Tetra Pak - Patent Attorneys SE
(86) International application number: PCT/EP2015/064707
(87) International publication number: WO 2016/001144

(56) References cited:
- EP-A1- 2 540 627
- JP-A- 2004 210 402
- US-A- 4 502 614
- US-A- 6 136 362

## Description

### Technical Field

The invention generally relates to the field of liquid or semi-liquid food processing systems. More particularly, it is presented a method for efficiently emptying a system with liquid product.

### Background of the invention

Today, it is in the interest of liquid food processing companies around the world to make sure that the food they are processing are done so in a way that assure that the food are safe to consume. This shows for instance in that each batch is carefully followed by using different kinds of sensors and sophisticated software tools, but also in that the design of the different components in the food processing lines are made in a way such that the risk that food residues are left from one batch to another is reduced to a very low likelihood.

Just as food safety is on top of the agenda for food processing companies, it is one of their top interests to make sure that product losses are kept at a minimum. Since the raw material, such as milk or fruit, in many cases has a significant role in the operational costs, lowering the product losses have in most cases a direct effect on the financial result. Secondly, in order to make sure that the processing is environmental friendly, the effect on e.g. carbon dioxide emissions can be significantly reduced if the losses of final product can be lowered.

One reason for having product losses is because the system is not emptied an efficient way. For example, if having the filling machine placed far from a buffer tank product in pipes connecting the filling machine and the tank, there is a risk that a substantial part of this product will be wasted. This problem is of course even bigger if small batches of different products are produced. Some prior art is reflected by patent documents US6136362A, EP2540627A1 and US4502614A.

### Summary

Accordingly, the present invention preferably seeks to mitigate, alleviate or eliminate one or more of the above-identified deficiencies in the art and disadvantages singly or in any combination and solves at least the above mentioned problems.

According to a first aspect it is provided a method for emptying a system filled with a liquid product, said system comprising an inlet, a tank, a first pipe connecting said inlet to said tank forming a first flow path, a second pipe connecting said tank to said inlet forming a second flow path, such that said first flow path and said second flow path provide a circulation flow path, wherein said second pipe is provided with a diversion valve such that at least part of said second flow path is diverted or that said second flow path is not diverted, wherein said second pipe is provided with a second valve placed between said tank and said diversion valve, a connection pipe connecting said second valve and a connection point of said second pipe placed between said diversion valve and said inlet, said method comprising feeding in sterile air into said tank such that liquid product in said tank and/or between said tank and said diversion valve is pushed out from said second pipe via said diversion valve, changing said second valve such that sterile air fed via said tank is directed to said connection point via said connection pipe, and feeding in sterile air into said tank such that liquid product in said tank and/or between said tank and said second valve and/or in said connection pipe and/or between said connection point and said diversion valve is pushed out from said second pipe via said diversion valve.

The system may further comprise a first valve provided on said first flow path, and said method may further comprise closing said first valve such that said first flow path is closed.

The tank may be provided with a tank inlet and a tank outlet, and said tank inlet may be placed above said tank outlet, and said step of closing said first valve such that said first flow path is closed may be performed if said tank is filled with product above said tank inlet.

The system may further comprise a first level sensor placed between said second valve and said diversion valve, and said method may further comprise detecting that a first amount of product in said second pipe between said second valve and said diversion valve is below a first threshold by using said first level sensor before changing said second valve such that sterile air fed via said tank is directed to said connection point.

The system may further comprise a second level sensor placed between said diversion valve and said connection point, and said method may further comprise detecting that a second amount of product in said second pipe between said connection point and said diversion valve is below a second threshold by using said second level sensor before ending said feeding of sterile air into said tank.

The product pushed out from said second pipe via said diversion valve is transferred to a filling machine.

According to a second aspect it is provided a system comprising an inlet, a tank provided with a sterile air inlet, a first pipe connecting said inlet to said tank forming a first flow path, a second pipe connecting said tank to said inlet forming a second flow path, such that said first flow path and said second flow path provide a circulation flow path, wherein said second pipe is provided with a diversion valve such that at least part of said second flow path is diverted or that said second flow path is not diverted, wherein said second pipe is provided with a second valve placed between said tank and said diversion valve, a connection pipe connecting said second valve to a connection point of said second pipe placed between said diversion valve and said inlet, such that by feeding in sterile air into said tank liquid product in said tank and/or between said tank and said diversion valve is pushed out from said second pipe via said diversion valve, and after changing said second valve such that sterile air fed via said tank is directed to said connection point by feeding in sterile air into said tank such that liquid product in said tank and/or between said tank and said second valve and/or in said connection pipe and/or between said connection point and said diversion valve is pushed out from said second pipe via said diversion valve.

A first valve may be provided on said first pipe such that before feeding in sterile air into said tank said first flow path can be closed by said first valve.

The tank may be provided with a tank inlet and a tank outlet, and said tank inlet may be placed above said tank outlet, and said system may be configured to close said first valve before feeding in sterile air into said tank if said tank is filled with product above said tank inlet.

A first level sensor may be placed between said second valve and said diversion valve.

A second level sensor placed between said diversion valve and said connection point.

The product pushed out from said second pipe via said diversion valve is transferred to a filling machine.

A length of a part of said second pipe connecting said connection point and said diversion valve may be longer than a length of a part of said second pipe connecting said connection point and said tank.

### Brief description of the drawings

The above, as well as additional objects, features and advantages of the present invention, will be better understood through the following illustrative and non-limiting detailed description of preferred embodiments of the present invention, with reference to the appended drawings, wherein:
Fig 1 generally illustrates an example of a downstream part of a food processing system including inter alia a tank and a filling machine.
Fig 2 generally illustrates an example of a downstream part of a food processing system including inter alia a tank and a filling machine, wherein said downstream part is provided with a circular connection.
Fig 3 is a general flow diagram illustrating another example of a downstream part of a food processing system including inter alia a tank and a filling machine, wherein said downstream part is provided with a circular connection.

### Detailed description of preferred embodiments

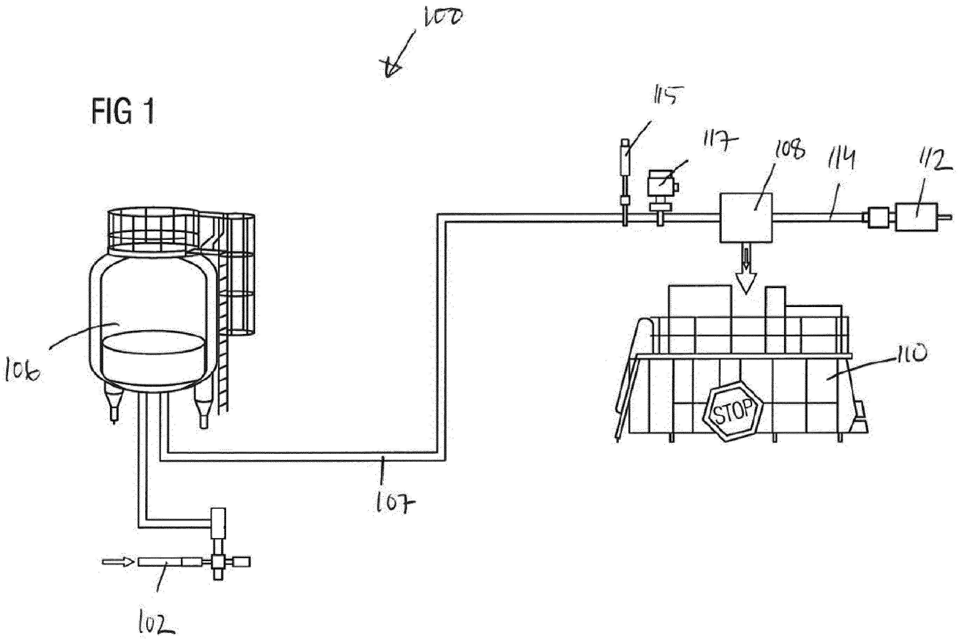

Fig 1 generally illustrates an example of a system 100 being part of a bigger system for processing and packing liquid or semi-liquid food products, such as so-called Extended Shelf Life (ESL) milk packed in carton packages. More particularly, the system 100 illustrates an inlet 102 for receiving heat treated product and feeding it to a tank 106, preferably a closed tank with a cooling system, arranged to hold the product without the risk of having it recontaminated. From the tank 106 the product is fed via a pipe 107 to a filling valve 108. The filling valve 108 can either direct the product to a filling machine 110 or to a reclaim tank via a reclaim valve 112 and a reclaim pipe 114, connecting the filling valve 108 and the reclaim valve 112.

During preparation of the system 100 product is fed via the inlet 102 to the tank 106 to form a buffer of product in the tank to balance out minor variations in the product flow upstream and downstream the tank 106. When having the buffer formed in the tank, the pipe 107 connecting the tank and the filling valve 108 is filled with product, and also the reclaim pipe 114.

After having filled the system 100 with product and it is made sure that temperature and pressure are within target intervals, for example by using a temperature sensor 115 and a pressure sensor 117, product is fed to the filling machine 110 where it is filled in packages.

During production, or in other words after having filled the system 100 and the product is fed to the filling machine 110, when handling ESL milk, the product should be kept at a temperature of about 4 degrees C to make sure that microbiological growth is prevented. If no product is fed to the reclaim tank for some time there is a risk that the product in the reclaim pipe 114 is heated, for instance by the surrounding environment, to a temperature above 4 degrees C. If this temperature increase is not prevented and kept under control, this may lead to microbacterial growth in the product kept in the reclaim pipe 114.

Further, if there is an unplanned stop of the filling machine, product will be standing in the pipe 107 connecting the tank 106 to the filling valve 108. If the stop lasts for a long period of time there is a risk of microbiological growth and, as an effect, that the product in the pipe 107 needs to be wasted and that the system needs to be cleaned before starting up again after the unplanned stop caused by the filling machine.

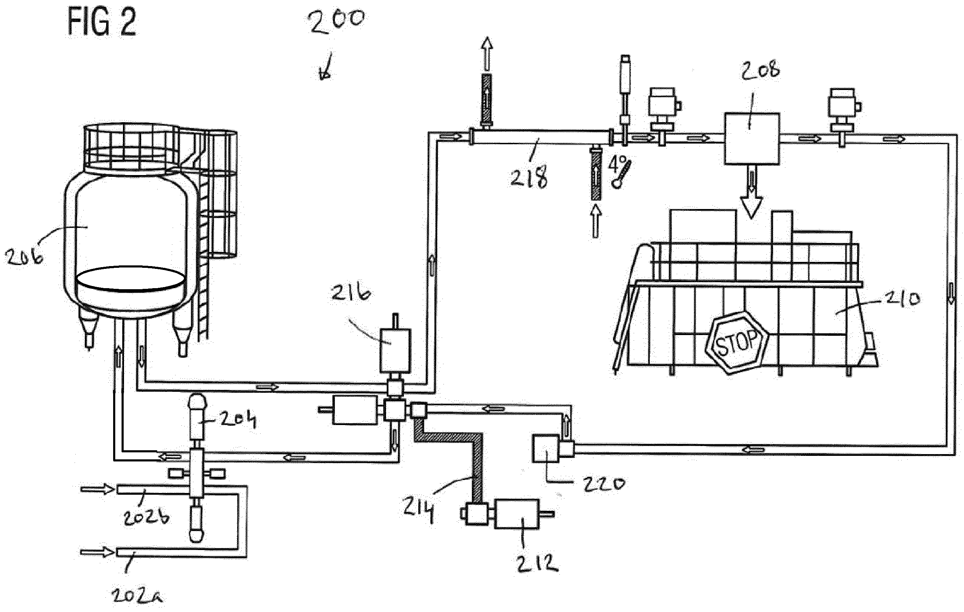

In order to overcome the problem of having product standing in the pipe 107 that may become bad in case of a long hault due to an unplanned stop caused by the filling machine 110, a system 200, illustrated in fig 2, can be used. Unlike the system 100 illustrated in fig 1, the system 200 provides for that the product can be circulated such that there will be no standing product in case of a filling machine stop.

The system 200 can comprise an inlet in turn comprising a first inlet 202a and a second inlet 202b, a valve 204 connected to to the inlet, a tank 206 forming a buffer to balance out variations in the product flow upstream and downstream, a filling valve 208 feeding part of a product flow to a filling machine 210 or in case the filling machine stops providing for that the product flow is fed pass the filling machine, a reclaim valve 212, a reclaim pipe 214 connecting the reclaim valve 212 to a valve arrangement 216, and an inline cooler 218 providing for that the product being circulated is kept within set temperature intervals.

During preparation sterile water is fed via the inlet. After having filled the system with sterile water and circulated the sterile water it can be fed out of the system to the drain via the reclaim valve 212.

If ESL milk or any other product that should be kept chilled is to be processed, the sterile water may be cold in order to make sure that the system is not only flushed, but also cooled before production starts.

After having had the system 200 flushed with sterile water, sterile air can be introduced into the system 200. Sterile air may be introduced via the tank 206 such that the sterile water present in the system is removed. This may for instance be done by introducing sterile air into the tank 206 and from there via the valve 204 to the valve arrangement 216 at the same time as sterile air is fed from the tank 206 via the valve arrangement 216, the inline cooler 218, the filling valve 208, the pump 220, back to the the valve arrangement 216, via the reclaim valve 212 to the reclaim tank.

After having flushed the system 200 with sterile water and sterile air, the system can be filled with product. This can be done by filling via the inlets 202a, 202b and feeding it via the valve 204, the tank 206, the valve arrangement 216, the inline cooler 218, the filling valve 208, the pump 220, the valve arrangement 216, the reclaim pipe 214, the reclaim valve 212 to the drain.

During production, product is fed via the inlets 202a, 202b via the valve 204, the tank 206, the valve arrangement 216, the inline cooler 218, the filling valve 208 to the filling machine 210. In order to make sure that no product is standing in the pipe connecting the filling valve 208 and the valve 204 part of the product may be diverted to the filling machine 210 and part of it circulated by feeding it back to the valve 204.

In case of an unplanned stop of the filling machine 210, or planned stop, the filling valve 208 can be changed such that all product are circulated and the incoming product result in that more product is buffered in the tank.

In order to make sure that the product is kept chilled, or in any other way within a target temperature interval, in case of a stop of the filing machine 210, the inline cooler 218 can be used. In the illustrated example, the inline cooler 218 is a tubular heat exchanger being provided with 4 degrees C water to make sure that a temperture of the product when passing the inline cooler is lowered to the target temperature interval. By doing so, the risk of having the product recontaminated during a stop of the filling machine 210 is reduced, which is positive from both a food safety perspective as well as a product loss perspective.

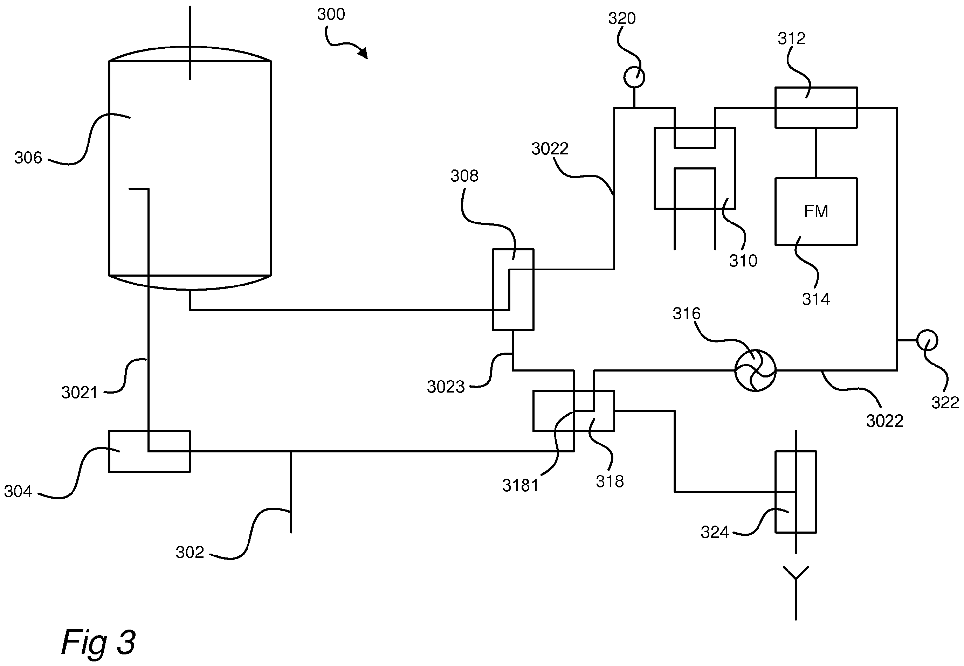

Fig 3 illustrates a flow diagram of a system 300 of a food processing system similar to the the system illustrated in fig 1 and 2.

In the system 300 liquid product can be fed via an inlet 302 via a first valve 304 to a tank 306. From the tank the liquid product can be fed via a second valve 308 and an inline cooler 310 to a diversion valve 312. The diversion valve 312 may be set to either divert part of a product flow to a filling machine 314 or not divert the flow to the filling machine such that the product flow is fed pass the filling machine 314. During production, the diversion valve can be set to divert part of the product flow. The reason why only part of the product flow is fed to the filling machine 314 is to make sure that there are no dead ends with standing product.

The product not diverted to the filling machine can be fed to a third valve 318 via a pump 316. From the third valve 318 the product may be fed to the inlet 302 such that the circular connection is achieved, providing for that the product can be circulated in the system. By having the inline cooler 310 in the system the product, when being recirculated, can be kept cool providing for that the risk for microbacterial growth can be kept low.

In order to keep track of the level in the system level sensors can be used. In the example illustrated in fig 3 a first level sensor 320 can be provided upstream the diversion valve 312 and a second level sensor 322 can be provided downstream the diversion valve 312.

The third valve 318 can be configured to either direct the product flow to the inlet as described above, or to direct the flow to a drain or reclaim system via a fifth valve 324. During cleaning, water and cleaning solutions can be sent to the drain or reclaim system.

After cleaning the fifth valve 324 may be set such that sterile air can be fed into the system via the fifth valve 324. In order to avoid that product is entering the part of the system between the fifth valve 324 and the third valve 318, herein referred to as a reclaim pipe, this part may be filled with overpressurized sterile air.

Sterile air may be used for emptying the tank 306. In case there is a system for providing sterile air to the tank this system may also be used for providing sterile air to the reclaim pipe.

An advantage of using overpressurized sterile air, that is having sterile air at a pressure in the reclaim pipe higher than a pressure in a closely placed pipe containing product during production, is that there is no need to use steam to ensure food safety. Unlike sterile air, steam will namely have the negative effect that product can burn on a part of the third valve being close to the reclaim pipe. Therefore, by having overpressurized sterile air instead of steam less fouling may occur, in turn resulting in improved running times.

When filling an empty system there may be air entrapped in the system. In order to make sure that this air is pushed out of the system and for instance not into the filling machine, the system can be filled with product by closing the first valve 304 and feeding in product via the inlet in a direction opposite to the direction of the product flow during production, namely via the third valve 318, the pump 316, the diversion valve 312, the second valve 308 to the tank 306. By using for instance a level switch in the tank 306, the system can be informed, by using a controller or other data handling apparatus, when the tank is filled to a certain level.

When the tank is filled to the certain level the first valve 304 can be opened such that product is fed from the inlet 302 via the first valve 304 to the tank 306.

In the illustrated example a first flow path from the inlet to the tank via the first valve is shorter than a second flow path from the inlet via the third valve, the pump, the diversion valve, the inline cooler, the second valve to the tank. Therefore, in the illustrated example, due to the first flow path is shorter than the second flow path, and that the second flow path is filled with product and the first flow path is empty, with the exception of the part between the inlet and the first valve, the product flow will choose the first flow path when opening the first valve. However, if needed to direct the product fed in via the inlet to the first flow path, a valve may be provided on the second flow path close to the inlet such that the second flow path can be closed.

After having filled the first flow path with product, product can be continued to be fed into the system via the inlet and the product flow from the tank can now switch to going from the tank via the second valve 308, the inline cooler 310 to the diversion valve 312, where part of the product flow can be fed to the filling machine 314 and part be fed via the pump 316, the third valve 318 and the first valve 304 to the tank 306.

In order to make sure that product losses can be kept low the system may be emptied by pushing out product present in the system by sterile air fed into the system via the tank 306.

In a first step of an emptying process, product placed in the system beween the tank, the second valve, the inline cooler and the diversion valve 312 can be pushed out to the filling machine by using sterile air fed into the system via the tank. In order to make sure that product is fed in this direction, the first valve 304 may be closed in this first step. Another option is, if having a tank inlet placed above a tank outlet, is to have the first valve closed when the tank level is above the tank inlet.

In a second step product placed in the system between the third valve 318, the pump 316 and the diversion valve 312 can be pushed out to the filling machine 314 by setting the second valve 308 such that sterile air fed from the tank is directed to the third valve 318. By emptying part of the system backwards in this way the product placed between the third valve and the diversion valve can be packed and used, instead of being sent to the drain. Since the distance between the diversion valve and the third valve may at some sites be long substantial savings can be made.

By having the first level sensor 320, an amount of product upstream the diversion valve 312 can be measured. When the amount of product is below a threshold the system can change such that product placed between the third valve 318 and the diversion valve 312 is pushed towards the diversion valve 312 by changing the second valve 308, as explained above.

Similarly, by having the second level sensor 322, an amount of product downstream the diversion valve can be measured in order to know when to stop feeding in sterile air in order to push product towards the diversion valve.

The invention has mainly been described above with reference to a few embodiments. However, as is readily appreciated by a person skilled in the art, other embodiments than the ones disclosed above are equally possible within the scope of the invention, as defined by the appended patent claims.

## Claims

1. A method for emptying a system filled with a liquid product, said system comprising an inlet (302), a tank (306), a first pipe (3021) connecting said inlet to said tank forming a first flow path, a second pipe (3022) connecting said tank (306) to said inlet (302) forming a second flow path, such that said first flow path and said second flow path provide a circulation flow path, wherein said second pipe (3022) is provided with a diversion valve (312) such that at least part of said second flow path is diverted or that said second flow path is not diverted, wherein said second pipe is provided with a second valve (308) placed between said tank (306) and said diversion valve (312), a connection pipe (3023) connecting said second valve (308) and a connection point (3181) of said second pipe (3022) placed between said diversion valve (312) and said inlet (302), said method comprising
feeding in sterile air into said tank (306) such that liquid product in said tank and/or between said tank (306) and said diversion valve (312) is pushed out from said second pipe (3022) via said diversion valve (312),
changing said second valve (308) such that sterile air fed via said tank (306) is directed to said connection point (3181) via said connection pipe (3023), and
feeding in sterile air into said tank (306) such that liquid product in said tank (306) and/or between said tank (306) and said second valve (308) and/or in said connection pipe (3023) and/or between said connection point (3181) and said diversion valve (312) is pushed out from said second pipe (3022) via said diversion valve (312), wherein
said product pushed out from said second pipe (3022) via said diversion valve (312) is transferred to a filling machine (314).

2. The method according to claim 1, wherein said system further comprising a first valve (304) provided on said first flow path (3021), said method further comprising
closing said first valve (304) such that said first flow path (3021) is closed.

3. The method according to claim 2, wherein said tank (306) is provided with a tank inlet and a tank outlet, and said tank inlet is placed above said tank outlet, said step of closing said first valve (304) such that said first flow path (3021) is closed is performed if said tank (306) is filled with product above said tank inlet.

4. The method according to any one of the preceding claims, wherein said system further comprising a first level sensor (320) placed between said second valve (308) and said diversion valve (312), said method further comprising
detecting that a first amount of product in said second pipe (3022) between said second valve (308) and said diversion valve (312) is below a first threshold by using said first level sensor (320) before changing said second valve (308) such that sterile air fed via said tank (306) is directed to said connection point (3181).

5. The method according to any one of the preceding claims, wherein said system further comprising a second level sensor (322) placed between said diversion valve (312) and said connection point (3181), said method further comprising
detecting that a second amount of product in said second pipe (3022) between said connection point (3181) and said diversion valve (312) is below a second threshold by using said second level sensor (322) before ending said feeding of sterile air into said tank (306).

6. A system comprising
an inlet (302),
a tank (306) provided with a sterile air inlet,
a first pipe (3021) connecting said inlet to said tank (306) forming a first flow path,
a second pipe (3022) connecting said tank (306) to said inlet (302) forming a second flow path,
such that said first flow path and said second flow path provide a circulation flow path,
wherein said second pipe (3022) is provided with a diversion valve (312) such that at least part of said second flow path is diverted or that said second flow path is not diverted,
wherein said second pipe (3022) is provided with a second valve (308) placed between said tank (306) and said diversion valve (312),
a connection pipe (3023) connecting said second valve (308) to a connection point (3181) of said second pipe (3022) placed between said diversion valve (312) and said inlet (302),
such that by feeding in sterile air into said tank (306) liquid product in said tank (306) and/or between said tank (306) and said diversion valve (312) is pushed out from said second pipe (3022) via said diversion valve (312), and after changing said second valve (308) such that sterile air fed via said tank (306) is directed to said connection point (3181) by feeding in sterile air into said tank (306) such that liquid product in said tank (306) and/or between said tank (306) and said second valve (308) and/or in said connection pipe (3023) and/or between said connection point (3181) and said diversion valve (312) is pushed out from said second pipe (3022) via said diversion valve (312), wherein
said product pushed out from said second pipe (3022) via said diversion valve (312) is transferred to a filling machine (314).

7. The system according to claim 6, further comprising a first valve (304) provided on said first pipe (3021) such that before feeding in sterile air into said tank (306) said first flow path can be closed by said first valve (304).

8. The system according to any one of the claims 6 or 7, wherein said tank (306) is provided with a tank inlet and a tank outlet, and said tank inlet is placed above said tank outlet, and said system is configured to close said first valve (304) before feeding in sterile air into said tank (306) if said tank (306) is filled with product above said tank inlet.

9. The system according to any one of the claims 6 to 8, further comprising a first level sensor (320) placed between said second valve (308) and said diversion valve (312).

10. The system according to any one of the claims 6 to 9, further comprising a second level sensor (322) placed between said diversion valve (312) and said connection point (3181).

11. The system according to any one of the claims 6 to 10, wherein a length of a part of said second pipe (3022) connecting said connection point (3181) and said diversion valve (312) is longer than a length of a part of said second pipe (3022) connecting said connection point (3181) and said tank (306).

## Patentansprüche

1. Verfahren zur Entleerung eines mit einem Flüssigprodukt gefüllten Systems, wobei das System einen Einlass (302), einen Tank (306), eine erste Leitung (3021), die den Einlass mit dem Tank verbindet, wobei eine erster Flusspfad gebildet wird, eine zweite Leitung (3022) umfasst, die den Tank (306) mit dem Einlass (302) verbindet, wobei ein zweiter Flusspfad gebildet wird, so dass der erste Flusspfad und der zweite Flusspfad einen Zirkulationsflusspfad bereitstellen, wobei die zweite Leitung (3022) mit einem Umleitventil (312) ausgestattet ist, so dass mindestens ein Teil des zweiten Flusspfades umgeleitet wird, oder dass der zweite Flusspfad nicht umgeleitet wird, wobei die zweite Leitung mit einem zweiten Ventil (308) ausgestattet ist, das zwischen dem Tank (306) und dem Umleitventil (312) platziert ist, wobei eine Verbindungsleitung (3023) das zweite Ventil (308) und einen Verbindungspunkt (3181) der zweiten Leitung (3022) verbindet, der zwischen dem Umleitventil (312) und dem Einlass (302) platziert ist, wobei das Verfahren umfasst:
Einspeisen von Sterilluft in den Tank (306), so dass Flüssigprodukt in dem Tank und/oder zwischen dem Tank (306) und dem Umleitventil (312) über das Umleitventil (312) aus der zweiten Leitung (3022) herausgedrückt wird,
Umschalten des zweiten Ventils (308), so dass über den Tank (306) eingespeiste Sterilluft über die Verbindungsleitung (3023) zu dem Verbindungspunkt (3181) geführt wird, und
Einspeisen von Sterilluft in den Tank (306), so dass Flüssigprodukt in dem Tank (306) und/oder zwischen dem Tank (306) und dem zweiten Ventil (308) und/oder in der Verbindungsleitung (3023) und/oder zwischen dem Verbindungspunkt (3181) und dem Umleitventil (312) über das Umleitventil (312) aus der zweiten Leitung (3022) herausgedrückt wird, wobei
das aus der zweiten Leitung (3022) über das Umleitventil (312) herausgedrückte Produkt in eine Füllmaschine (314) überführt wird.

2. Verfahren nach Anspruch 1, wobei das System ferner ein erstes Ventil (304) umfasst, das auf dem ersten Flusspfad (3021) bereitgestellt ist, wobei das Verfahren ferner umfasst
Schließen des ersten Ventils (304) derart, dass der erste Flusspfad (3021) verschlossen ist.

3. Verfahren nach Anspruch 2, wobei der Tank (306) mit einem Tankeinlass und einem Tankauslass ausgestattet ist, und wobei der Tankeinlass oberhalb des Tankauslasses platziert ist, wobei der Schritt des Schließens des ersten Ventils (304), so dass der erste Flusspfad (3021) geschlossen ist, durchgeführt wird, wenn der Tank (306) mit Produkt oberhalb des Tankeinlasses gefüllt ist.

4. Verfahren nach einem der vorhergehenden Ansprüche, wobei das System ferner einen ersten Pegelsensor (320) umfasst, der zwischen dem zweiten Ventil (308) und dem Umleitventil (312) platziert ist, wobei das Verfahren ferner umfasst:
Detektieren, dass eine erste Menge an Produkt in der zweiten Leitung (3022) zwischen dem zweiten Ventil (308) und dem Umleitventil (312) unterhalb eines ersten Schwellenwerts ist, indem der erste Pegelsensor (320) verwendet wird, bevor das zweite Ventil (308) umgeschaltet wird, so dass über den Tank (306) eingespeiste Sterilluft zu dem Verbindungspunkt (3181) gelenkt wird.

5. Verfahren nach einem der vorhergehenden Ansprüche, wobei das System ferner einen zweiten Pegelsensor (322) umfasst, der zwischen dem Umleitventil (312) und dem Verbindungspunkt (3181) platziert ist, wobei das Verfahren ferner umfasst:
Detektieren, dass eine zweite Menge an Produkt in der zweiten Leitung (3022) zwischen dem Verbindungspunkt (3181) und dem Umleitventil (312) unterhalb eines zweiten Schwellenwerts ist, indem der zweite Pegelsensor (322) verwendet wird, bevor das Einspeisen von Sterilluft in den Tank (306) beendet wird.

6. System, umfassend:
einen Einlass (302),
einen Tank (306), der mit einem Sterillufteinlass ausgestattet ist,
eine erste Leitung (3021), die den Einlass mit dem Tank (306) verbindet, wodurch ein erster Flusspfad gebildet wird,
eine zweite Leitung (3022), die den Tank (306) mit dem Einlass (302) verbindet, wodurch ein zweiter Flusspfad gebildet wird,
so dass der erste Flusspfad und der zweite Flusspfad einen Zirkulationsflusspfad bereitstellen,
wobei die zweite Leitung (3022) mit einem Umleitventil (312) ausgestattet ist, so dass mindestens ein Teil des zweiten Flusspfades umgeleitet wird oder dass der zweite Flusspfad nicht umgeleitet wird,
wobei die zweite Leitung (3022) mit einem zweiten Ventil (308) ausgestattet ist, das zwischen dem Tank (306) und dem Umleitventil (312) platziert ist,
eine Verbindungsleitung (3023), die das zweite Ventil (308) mit einem Verbindungspunkt (3181) der zweiten Leitung (3022) verbindet, der zwischen dem Umleitventil (312) und dem Einlass (302) platziert ist,
so dass durch Einspeisen von Sterilluft in den Tank (306) Flüssigprodukt in dem Tank (306) und/oder zwischen dem Tank (306) und dem Umleitventil (312) über das Umleitventil (312) aus der zweiten Leitung (3022) herausgedrückt wird, und nach Umschalten des zweiten Ventils (308), so dass über den Tank (306) eingespeiste Sterilluft zu dem Verbindungspunkt (3181) gelenkt wird, indem Sterilluft in den Tank (306) eingespeist wird, so dass Flüssigprodukt in dem Tank (306) und/oder zwischen dem Tank (306) und dem zweiten Ventil (308) und/oder in der Verbindungsleitung (3023) und/oder zwischen dem Verbindungspunkt (3181) und dem Umleitventil (312) über das Umleitventil (312) aus der zweiten Leitung (3022) herausgedrückt wird, wobei
das aus der zweiten Leitung (3022) über das Umleitventil (312) herausgedrückte Produkt in eine Füllmaschine (314) überführt wird.

7. System nach Anspruch 6, ferner umfassend ein erstes Ventil (304), das auf der ersten Leitung (3021) bereitgestellt wird, so dass der erste Flusspfad vor dem Einspeisen von Sterilluft in den Tank (306) durch das erste Ventil (304) geschlossen werden kann.

8. System nach einem der Ansprüche 6 oder 7, wobei der Tank (306) mit einem Tankeinlass und einem Tankauslass ausgestattet ist, und wobei der Tankeinlass oberhalb des Tankauslasses platziert ist, und wobei das System ausgestaltet ist, um das erste Ventil (304) zu schließen, bevor Sterilluft in den Tank (306) eingespeist wird, wenn der Tank (306) mit Produkt oberhalb des Tankeinlasses gefüllt ist.

9. System nach einem der Ansprüche 6 bis 8, ferner umfassend einen ersten Pegelsensor (320), der zwischen dem zweiten Ventil (308) und dem Umleitventil (312) platziert ist.

10. System nach einem der Ansprüche 6 bis 9, ferner umfassend einen zweiten Pegelsensor (322), der zwischen dem Umleitventil (312) und dem Verbindungspunkt (3181) platziert ist.

11. System nach einem der Ansprüche 6 bis 10, wobei eine Länge eines Teils der zweiten Leitung (3022), die den Verbindungspunkt (3181) und das Umleitventil (312) verbindet, länger als eine Länge eines Teils der zweiten Leitung (3022) ist, die den Verbindungspunkt (3181) und den Tank (306) verbindet.

## Revendications

1. Procédé pour vider un système rempli avec un produit liquide, ledit système comprenant une entrée (302), un réservoir (306), un premier tuyau (3021) qui connecte ladite entrée audit réservoir, formant un premier chemin d'écoulement, un second tuyau (3022) qui connecte ledit réservoir (306) à ladite entrée (302), formant un second chemin d'écoulement, de telle sorte que ledit premier chemin d'écoulement et ledit second chemin d'écoulement forment un chemin d'écoulement de circulation, dans lequel ledit second tuyau (3022) est pourvu d'une soupape de déviation (312) de telle sorte qu'au moins une partie dudit second chemin d'écoulement soit déviée ou que ledit second chemin d'écoulement ne soit pas dévié, dans lequel ledit second tuyau est pourvu d'une seconde soupape (308) qui est placée entre ledit réservoir (306) et ladite soupape de déviation (312), un tuyau de connexion (3023) connectant ladite seconde soupape (308) et un point de connexion (3181) dudit second tuyau (3022) placé entre ladite soupape de déviation (312) et ladite entrée (302), ledit procédé comprenant les étapes suivantes:
introduire de l'air stérile dans ledit réservoir (306) de telle sorte qu'un produit liquide dans ledit réservoir et/ou entre ledit réservoir (306) et ladite soupape de déviation (312) soit poussé hors dudit second tuyau (3022) par l'intermédiaire de ladite soupape de déviation (312),
changer ladite seconde soupape (308) de telle sorte que l'air stérile fourni par l'intermédiaire dudit réservoir (306) soit dirigé vers ledit point de connexion (3181) par l'intermédiaire dudit tuyau de connexion (3023), et
introduire de l'air stérile dans ledit réservoir (306) de telle sorte qu'un produit liquide dans ledit réservoir (306) et/ou entre ledit réservoir (306) et ladite seconde soupape (308) et/ou dans ledit tuyau de connexion (3023) et/ou entre ledit point de connexion (3181) et ladite soupape de déviation (312) soit poussé hors dudit second tuyau (3022) par l'intermédiaire de ladite soupape de déviation (312),
dans lequel ledit produit poussé hors dudit second tuyau (3022) par l'intermédiaire de ladite soupape de déviation (312) est transféré à une machine de remplissage (314).

2. Procédé selon la revendication 1, dans lequel ledit système comprend en outre une première soupape (304) prévue sur ledit premier chemin d'écoulement (3021), ledit procédé comprenant en outre la fermeture de ladite première soupape (304) de telle sorte que ledit premier chemin d'écoulement (3021) soit fermé.

3. Procédé selon la revendication 2, dans lequel ledit réservoir (306) est pourvu d'une entrée de réservoir et d'une sortie de réservoir, et ladite entrée de réservoir est placée au-dessus de ladite sortie de réservoir, ladite étape de fermeture de ladite première soupape (304) selon laquelle ledit premier chemin d'écoulement (3021) est fermé est exécutée si ledit réservoir (306) est rempli avec un produit au-dessus de ladite entrée de réservoir.

4. Procédé selon l'une quelconque des revendications précédentes, dans lequel ledit système comprend en outre un premier capteur de niveau (320) qui est placé entre ladite seconde soupape (308) et ladite soupape de déviation (312), ledit procédé comprenant en outre la détection qu'une première quantité de produit dans ledit second tuyau (3022) entre ladite seconde soupape (308) et ladite soupape de déviation (312) est inférieure à un premier seuil en utilisant ledit première capteur de niveau (320) avant le changement de ladite seconde soupape (308) de telle sorte que l'air stérile fourni par l'intermédiaire dudit réservoir (306) soit dirigé vers ledit point de connexion (3181).

5. Procédé selon l'une quelconque des revendications précédentes, dans lequel ledit système comprend en outre un second capteur de niveau (322) qui est placé entre ladite soupape de déviation (312) et ledit point de connexion (3181), ledit procédé comprenant en outre la détection qu'une seconde quantité de produit dans ledit second tuyau (3022) entre ledit point de connexion (3181) et ladite soupape de déviation (312) est inférieure à un second seuil en utilisant ledit seconde capteur de niveau (322) avant de terminer l'introduction d'air stérile dans ledit réservoir (306) .

6. Système, comprenant
une entrée (302),
un réservoir (306) pourvu d'une entrée d'air stérile,
un premier tuyau (3021) qui connecte ladite entrée audit réservoir (306), formant un premier chemin d'écoulement,
un second tuyau (3022) qui connecte ledit réservoir (306) à ladite entrée (302), formant un second chemin d'écoulement,
de telle sorte que ledit premier chemin d'écoulement et ledit second chemin d'écoulement forment un chemin d'écoulement de circulation,
dans lequel ledit second tuyau (3022) est pourvu d'une soupape de déviation (312) de telle sorte qu'au moins une partie dudit second chemin d'écoulement soit déviée ou que ledit second chemin d'écoulement ne soit pas dévié,
dans lequel ledit second tuyau (3022) est pourvu d'une seconde soupape (308) qui est placée entre ledit réservoir (306) et ladite soupape de déviation (312),
un tuyau de connexion (3023) qui connecte ladite seconde soupape (308) à un point de connexion (3181) dudit second tuyau (3022) placé entre ladite soupape de déviation (312) et ladite entrée (302),
de telle sorte que, en introduisant de l'air stérile dans ledit réservoir (306), un produit liquide dans ledit réservoir (306) et/ou entre ledit réservoir (306) et ladite soupape de déviation (312) soit poussé hors dudit second tuyau (3022) par l'intermédiaire de ladite soupape de déviation (312) et, après le changement de ladite seconde soupape (308), de telle sorte que l'air stérile fourni par l'intermédiaire dudit réservoir (306) soit dirigé vers ledit point de connexion (3181) en introduisant de l'air stérile dans ledit réservoir (306) de telle sorte qu'un produit liquide dans ledit réservoir (306) et/ou entre ledit réservoir (306) et ladite seconde soupape (308) et/ou dans ledit tuyau de connexion (3023) et/ou entre ledit point de connexion (3181) et ladite soupape de déviation (312) soit poussé hors dudit second tuyau (3022) par l'intermédiaire de ladite soupape de déviation (312),
dans lequel ledit produit sortant dudit second tuyau (3022) par l'intermédiaire de ladite soupape de déviation (312) est transféré à une machine de remplissage (314).

7. Système selon la revendication 6, comprenant en outre une première soupape (304) prévue sur ledit premier tuyau (3021) de telle sorte qu'avant d'introduire de l'air stérile dans ledit réservoir (306), ledit premier chemin d'écoulement puisse être fermé par ladite première soupape (304).

8. Système selon l'une quelconque des revendications 6 ou 7, dans lequel ledit réservoir (306) est pourvu d'une entrée de réservoir et d'une sortie de réservoir, et ladite entrée de réservoir est placée au-dessus de ladite sortie de réservoir, et ledit système est configuré de manière à fermer ladite première soupape (304) avant d'introduire de l'air stérile dans ledit réservoir (306) si ledit réservoir (306) est rempli avec un produit au-dessus de ladite entrée de réservoir.

9. Système selon l'une quelconque des revendications 6 à 8, comprenant en outre un premier capteur de niveau (320) qui est placé entre ladite seconde soupape (308) et ladite soupape de déviation (312).

10. Système selon l'une quelconque des revendications 6 à 9, comprenant en outre un second capteur de niveau (322) qui est placé entre ladite soupape de déviation (312) et ledit point de connexion (3181).

11. Système selon l'une quelconque des revendications 6 à 10, dans lequel une longueur d'une partie dudit second tuyau (3022) qui connecte ledit point de connexion (3181) et ladite soupape de déviation (312) est plus longue qu'une longueur d'une partie dudit second tuyau (3022) qui connecte ledit point de connexion (3181) et ledit réservoir (306).
